# EUROPEAN PATENT APPLICATION

(11) **EP 1 413 310 A1**
(43) Date of publication of application: **28.04.2004**
(21) Application number: 02741445.7
(22) Date of filing: 08.07.2002
(51) Int. Cl.: A61K 38/21, A61K 38/00, A61K 9/14, A61K 9/56, A61K 47/26, A61K 47/34, A61K 47/40, A61K 47/44, A61P 43/00

(54) **SUSTAINED-RELEASE COMPOSITIONS FOR INJECTION AND PROCESS FOR PRODUCING THE SAME**

(30) Priority: 09.07.2001 JP 2001207833
(71) Applicant: YAMANOUCHI PHARMACEUTICAL CO. LTD., Tokyo 103-8411 (JP)
(72) Inventor: MAEDA, Atsushi, Yaizu-shi, Shizuoka 425-0072 (JP); TAKAISHI, Yuuki, Yaizu-shi, Shizuoka 425-0072 (JP); NAKANISHI, Kiyo, Yaizu-shi, Shizuoka 425-0072 (JP); SAITO, Katsumi, Yaizu-shi, Shizuoka 425-0072 (JP); YAMASHITA, Noboru, Yaizu-shi, Shizuoka 425-0072 (JP); TAKAGI, Akira, Yaizu-shi, Shizuoka 425-0072 (JP)
(74) Representative: Bates, Philip Ian
(86) International application number: PCT/JP2002/006903
(87) International publication number: WO 2003/006052

(57) **Abstract**

This invention relates to the provision of a composition for sustained release injections, wherein a physiologically active protein is spray-dried while keeping its physiological activity, this is incorporated in a biodegradable polymer with a high efficiency, and reduction of its initial burst and continuous dissolution thereof for a prolonged period of time are attained. Also, the invention relates to a physiologically active protein-containing composition for sustained release injections, which comprises a physiologically active protein and a saccharide as substantial composing components, wherein a spray-dried fine powder, 90% or more of which has a particle diameter of 10 µm or less, is dispersed in an organic solvent solution of a biodegradable polymer, and said organic solvent is removed in an oil.

## Description

### Technical Field

This invention relates to a composition for sustained release injections, in which a physiologically active protein is incorporated in a biodegradable polymer, and to a production method thereof. More particularly, it relates to a physiologically active protein-containing composition for sustained release injections, in which a spray-dried fine powder, of which particles comprising a physiologically active protein and a saccharide, and 90% or more of which particles are 10µm or less, is uniformly dispersed in the matrix of a biodegradable polymer.

### Background of the Invention

Administration of physiologically active proteins to human has been broadly carried out, but since oral absorption of proteins are generally low and their disappearance from the living body is quick, it is the current situation that they are frequently administered as injections. Under such circumstances, a sustained release technique for preparing a microsphere by incorporating a physiologically active protein in a biodegradable polymer is strongly in demand from the viewpoint of avoiding frequent administration and improving therapeutic effect. In addition, since proteins undergo denaturation by heat, solvent and the like, difficulty in their handling from the production point of view has been pointed out and its avoiding method has been expected. However, virtually nothing is known about a sustained release microsphere production technique which can incorporate a physiologically active protein in a microsphere stably and with a high encapsulation ratio, reduces rapid release of the physiologically active protein at an initial stage after commencement of a dissolution test (to be referred to as initial burst hereinafter) and has productivity and practicability.

In order to avoid reduction of the physiological activity of a physiologically active protein, attempts have been made to keep the activity by making the water-soluble protein into powder for the purpose of avoiding its denaturation by an organic solvent (e.g., methylene chloride and the like). For example, JP-A-11-322631 discloses a sustained release preparation of a physiologically active polypeptide, in which stable incorporation of a polypeptide in the preparation was achieved by adding a water-miscible organic solvent and/or a volatile salt (e.g., an ammonium salt or the like) to a physiologically active polypeptide aqueous solution and then making the solution into powder by freeze drying technique , and thereby its encapsulation ratio and sustained release property are improved. In addition, JP-A-11-302156 discloses that polypeptide fine particles with high purity are prepared with high recovery yield without accompanying denaturation, by freeze-drying a mixed solution of a polypeptide and polyethylene glycol and by using a solvent in which the polypeptide cannot be dissolved but polyethylene glycol and a biodegradable polymer can be dissolved, and thereby fine particles are incorporated in a microsphere. These techniques are relating to the freeze-drying techniques for making fine particles of polypeptides followed by the microsphere preparation and not relating to a spray-drying technique.

A technique which uses spray drying followed by the microsphere preparation is disclosed in JP-A-4-36233. Said technique relates to a method which attained sustained release for a prolonged period of time, increment of encapsulation ratio of a drug, and reduction of initial burst, by spray-drying a mixed solution of a polypeptide and a water-soluble polymer such as gelatin or the like, thereby making the solution into fine particles, and further coating them with a biodegradable polymer solution. However, temperature for the spray drying sometimes becomes high depending on the water-soluble polymer to be used, thus a possibility of polypeptide inactivation is concerned.

Virtually nothing is known about techniques which can achieve sufficient effects in terms of the keeping of physiological activity as well as control of initial release, high incorporation ratio of physiologically active protein, continuous releasing for a prolonged period of time, productivity and practicality, so that there is room for further improving them to satisfy all of these requirements.

Accordingly, the object of the invention is to provide physiologically active protein-containing sustained release injections, which are prepared by making a physiologically active protein into fine powder through its spray drying while keeping its physiological activity and incorporating it in a biodegradable polymer with a high incorporation ratio, and which also keeps physiological activity of the physiologically active protein in that case, can attain reduction of initial burst and continuous releasing for a prolonged period of time, and has high productivity and practicality.

### Disclosure of the Invention

Under such circumstances, the present inventors have examined various techniques for making a physiologically active protein into fine powder by spray drying and found that the fine powder having proper particle diameter can be obtained by the spray drying without causing denaturation of the physiologically active protein when a saccharide, particularly mannitol or 2-hydroxypropyl-β-cyclodextrin, is used as a carrier, and that when a method is employed in which fine powder of the thus obtained physiologically active protein is incorporated in a sustained release base material comprising a biodegradable polymer in an oil, the physiological activity is completely remained and high inclusion ratio, reduction of initial burst and continuous drug release for a prolonged period of time can be attained, thereby resulting in the accomplishment of the invention.

According to the invention, spray drying can be carried out without increasing the drying temperature by the use of saccharides, so that denaturation and inactivation of a physiologically active protein by heat can be avoided and preparation of a protein fine powder having controlled particle diameter becomes markedly easy. Also, the use of such a protein fine powder which keeps the physiological activity and has a sufficiently controlled particle diameter renders possible keeping of physiological activity of the physiologically active protein incorporated in microspheres and attainment of high encapsulation ratio into microsphere and initial burst reduction.

That is, the present invention relates to,
1. a physiologically active protein-containing composition for sustained release injections, which comprises a spray-dried fine powder comprising a physiologically active protein and a saccharide or the like, wherein particles having a particle diameter of 10 µm or less occupy 90% or more of the spray-dried fine powder, and the spray-dried fine powder is uniformly dispersed in the matrix of a biodegradable polymer,
2. the physiologically active protein-containing composition for sustained release injections described in the aforementioned item 1, wherein 50% or more of a spray-dried fine powder has a particle diameter of 6 µm or less,
3. the physiologically active protein-containing composition for sustained release injections described in the aforementioned item 2, wherein the saccharide is mannitol and/or a cyclodextrin or the like,
4. the physiologically active protein-containing composition for sustained release injections described in the aforementioned item 3, wherein the cyclodextrin is 2-hydroxypropyl-β-cyclodextrin,
5. the physiologically active protein-containing composition for sustained release injections described in the aforementioned item 4, wherein the biodegradable polymer is a poly (lactic acid-glycolic acid) copolymer,
6. the physiologically active protein-containing composition for sustained release injections described in the aforementioned item 5, wherein the physiologically active protein is an interferon or the like,
7. the physiologically active protein-containing composition for sustained release injections described in the aforementioned item 6, wherein the interferon is interferon-α-Con 1,
8. the physiologically active protein-containing composition for sustained release injections described in the aforementioned item 7, wherein the composition is microsphere,
9. the physiologically active protein-containing composition for sustained release injections described in the aforementioned item 8, wherein average particle diameter of the microsphere is set to from about 20 to about 250 µm,
10. the physiologically active protein-containing composition for sustained release injections described in the aforementioned item 9, wherein weight ratio of the physiologically active protein and the saccharide is from about 0.01 to about 80% (w/w),
11. the physiologically active protein-containing composition for sustained release injections described in the aforementioned item 9, wherein concentration of the spray-dried fine powder in the biodegradable polymer is from about 0.01 to about 20% (w/w),
12. a physiologically active protein-containing composition for sustained release injections, which comprises a physiologically active protein and a saccharide as substantial composition components, and is prepared by dispersing a spray-dried fine powder, 90% or more of which has a particle diameter of 10 µm or less, in an organic solvent solution of a biodegradable polymer, and removing said organic solvent in an oil,
13. the physiologically active protein-containing composition for sustained release injections described in the aforementioned item 12, wherein 50% or more of a spray-dried fine powder has a particle diameter of 6 µm or less,
14. the physiologically active protein-containing composition for sustained release injections described in the aforementioned item 13, wherein the saccharide is mannitol and/or a cyclodextrin or the like,
15. the physiologically active protein-containing composition for sustained release injections described in the aforementioned item 14, wherein the cyclodextrin is 2-hydroxypropyl-β-cyclodextrin,
16. the physiologically active protein-containing composition for sustained release injections described in the aforementioned item 15, wherein the biodegradable polymer is a poly (lactic acid-glycolic acid) copolymer,
17. the physiologically active protein-containing composition for sustained release injections described in the aforementioned item 16, wherein the physiologically active protein is an interferon or the like,
18. the physiologically active protein-containing composition for sustained release injections described in the aforementioned item 17, wherein the interferon is interferon-α-Con 1,
19. the physiologically active protein-containing composition for sustained release injections described in the aforementioned item 18, wherein the oil is silicone oil, soybean oil, sesame oil, cotton seed oil or mineral oil,
20. the physiologically active protein-containing composition for sustained release injections described in the aforementioned item 19, wherein the oil is silicone oil,
21. the physiologically active protein-containing composition for sustained release injections described in the aforementioned item 20, wherein the composition is microsphere,
22. the physiologically active protein-containing composition for sustained release injections described in the aforementioned item 21, wherein average particle diameter of the microsphere is from about 20 to about 250 µm,
23. the physiologically active protein-containing composition for sustained release injections described in the aforementioned item 9, wherein weight ratio of the physiologically active protein and the saccharide is from about 0.01 to about 80% (w/w),
24. the physiologically active protein-containing composition for sustained release injections described in the aforementioned item 22, wherein concentration of the spray-dried fine powder in the biodegradable polymer is 10% (w/w) or less,
25. a method for producing a physiologically active protein-containing composition for sustained release injections, which comprises a physiologically active protein and a saccharide as substantial composition components, and which comprises using dispersion of a spray-dried fine powder, 90% or more of which being occupied by particles having a particle diameter of 10 µm or less, in an organic solvent solution of a biodegradable polymer, and removing said organic solvent in an oil,
26. the method for producing a physiologically active protein-containing composition for sustained release injections described in the aforementioned item 25, wherein 50% on more of a spray-dried fine powder has a particle diameter of 6 µm or less,
27. the method for producing a physiologically active protein-containing composition for sustained release injections described in the aforementioned item 26, wherein the saccharide is mannitol and/or a cyclodextrin or the like,
28. the method for producing a physiologically active protein-containing composition for sustained release injections described in the aforementioned item 27, wherein the cyclodextrin is 2-hydroxypropyl-β-cyclodextrin,
29. the method for producing a physiologically active protein-containing composition for sustained release injections described in the aforementioned item 28, wherein the biodegradable polymer is a poly (lactic acid-glycolic acid) copolymer,
30. the method for producing a physiologically active protein-containing composition for sustained release injections described in the aforementioned item 29, wherein the physiologically active protein is an interferon or the like,
31. the method for producing a physiologically active protein-containing composition for sustained release injections described in the aforementioned item 30, wherein the interferon is interferon-α-Con 1,
32. the method for producing a physiologically active protein-containing composition for sustained release injections described in the aforementioned item 31, wherein the oil is silicone oil, soybean oil, sesame oil, cotton seed oil or mineral oil,
33. the method for producing a physiologically active protein-containing composition for sustained release injections described in the aforementioned item 32, wherein the oil is silicone oil,
34. the method for producing a physiologically active protein-containing composition for sustained release injections described in the aforementioned item 33, wherein the composition is microsphere,
35. the method for producing a physiologically active protein-containing composition for sustained release injections described in the aforementioned item 34, wherein average particle diameter of the microsphere is from about 20 to about 250 µm,
36. the method for producing a physiologically active protein-containing composition for sustained release injections described in the aforementioned item 35, wherein concentration of the spray-dried fine powder in the biodegradable polymer is 10% (w/w) or less,
37. the method for producing a physiologically active protein-containing composition for sustained release injections described in the aforementioned item 9, wherein weight ratio of the physiologically active protein and the saccharide is from about 0.01 to about 80% (w/w).

The term "matrix" as used herein means a network structure of a biodegradable polymer. Also, the term "uniformly dispersed" means a state in which a spray-dried powder comprising a physiologically active protein and a saccharide is sufficiently dispersed in the network structure of a biodegradable polymer, and in case that the initial burst does not occur when release profile of the composition of the invention is measured by a dissolution test which will be described later, it can be known indirectly that the physiologically active protein is "uniformly dispersed".

Also, significance of the measurement of "particle diameter of a spray-dried fine powder" according to the invention is to understand a particle diameter which facilitates its incorporation in a biodegradable polymer and renders possible attainment of initial burst reduction and high incorporation ratio, and illustratively, it is desirable that 90% or more of powder has a particle diameter of 10 µm or less, it is more desirable that 50% or more of powder has a particle diameter of 6 µm or less. The use of such a fine powder having even-sized particle facilitates the subsequent inclusion of a spray-dried fine powder, namely a physiologically active protein, in a biodegradable polymer and renders possible attainment of initial burst reduction and high encapsulation ratio.

The term "microsphere" as used herein means a spherical particle having a particle diameter of from several ten µm to several hundred µm, and illustratively, it means a spherical particle of from about 10 µm to about 300 µm, preferably from about 20 µm to about 250 µm.

The term "comprises a physiologically active protein and a saccharide as substantial composition components" as used herein means that a physiologically active protein and a saccharide are used as the main components but other components such as an excipient and the like may also be contained in case that such components do not damage the effect of the current invention. Also, the term "keeping of physiological activity" of a physiologically active protein as used herein means that when titer of a physiologically active protein is measured by, for example, the physiological activity testing method shown in the following, the titer of a sample based on a standard preparation is from about 70 to about 125%, preferably from about 80 to about 115%.

In addition, the terms "reduction of initial burst" and "continuous release" in the physiologically active protein-incorporated composition for sustained release injections of the invention mean that the "reduction of initial burst" is release rate of about 10% or less in 3 hours after commencement of the test and "continuous release" means "sustained release property" and illustratively means that release of a drug continues for a period of approximately from 1 day to 1 month after commencement of the test.

Still more, the term "high encapsulation ratio" of a physiologically active protein as used herein means that, when measured by the encapsulation ratio measuring method shown in the following, it shows an encapsulation ratio of about 70% or more, preferably about 75% or more, more preferably about 80% or more.

In addition, the term "without increasing " of the drying temperature at the time of spray drying means a low temperature range of the outlet temperature when a spray drier for example is selected as the apparatus, and it illustratively means a range of from about 0°C to about 80°C, preferably from about 10°C to about 60°C, more preferably from about 20°C to about 60°C.

The physiologically active protein to be used in the invention is not particularly limited, with the proviso that it is a protein to be used as a therapeutically active component. Examples of such a therapeutically active component include a cytokine, a growth factor, a hematopoietic factor, a peptide hormone, an enzyme and the like.

Illustratively, interferons (α type, β type, γ type and the like), interleukins (IL-1, 2, 3, 4, 5, 6, 7, 8, 9, 10, 11, 12, 13, 14, 15, 16, 17, 18, 19, 20, 21, 22, 23 and the like), tumor necrosis factor (TNF) and the like can be exemplified as the cytokine, and bone morphogenetic protein (BMP-1, 2, 3, 4 and the like), nerve growth factors (NGF-1, 2 and the like), neurotrophic factor (NTF), epidermal growth factor (EGF), insulin-like growth factors (IGF-1, 2, 3 and the like), fibroblast growth factors (aFGF and bFGF), platelet-derived growth factor (PDGF), cartilage-derived factor (CDF), transforming growth factors (TGF-α, -β and the like) and the like can be exemplified as the growth factor.

Also, colony-stimulating factors (G-, M-, GM-CSF and the like), thrombopoietin (TPO), erythropoietin (EPO), platelet growth stimulation factor and the like can be exemplified as the hematopoietic factor, and growth hormone (GH), growth hormone-releasing factor (GRF), adenocorticotropic hormone (ACTH), thyroid-stimulating hormone (TSH), follicle-stimulating hormone (FSH), luteinizing hormone (LH), human cilium gonadotropin (hCG), melanocyte-stimulating hormone (MSH), insulin, glucagon, prolactin, calcitonin, natriuretic peptide and the like as the peptide hormone, and tissue plasminogen activator (tPA), urokinase (UK), superoxide dismutase (SOD), asparaginase, kallikrein, streptokinase, protein C, metalloproteases, factor VIII and factor IX, puruoxidase and the like as the enzyme.

Preferred as the physiologically active protein of the invention are interferons, bone morphogenetic proteins and growth hormones, more preferred are interferons and growth hormones, and most preferred are interferons. The interferons include both of natural types and recombinant types. Among them, a recombinant type interferon-α-Con 1 is particularly desirable. These physiologically active proteins may be used alone or as a combination of two or more. In addition, a combination with a non-protein drug such as an antiviral agent or the like can be employed.

The physiologically active protein to be used in the invention has a molecular weight of from about 1,000 to about 200,000, preferably from about 5,000 to about 70,000. These physiologically active proteins may be natural origins or modified products obtained by genetic recombination techniques and the like, or may be modified products thereof (e.g., a chemically modified product with polyethylene glycol or the like). Also, these may be used as monomers or as homo or hetero multimeric form of protein.

The saccharides to be used in the invention means cyclic structure and (linear chain and branched chain) monosaccharides, disaccharides, oligosaccharides, polysaccharides, derivatives thereof (including sugar alcohols and the like) and the like can be exemplified, of which sugar alcohols or cyclic oligosaccharides are desirable, mannitol or cyclodextrins (α type, β type, γ type, 2-hydroxypropyl-β-cyclodextrin and the like) are more desirable, and mannitol and 2-hydroxypropyl-β-cyclodextrin are most desirable. In addition, these saccharides may be used alone or as a combination of two or more.

The biodegradable polymer according to the invention means a polymer which has biocompatibility but does not have physiological activity and is degraded and cleared from the living body, and is not particularly limited with the proviso that it is pharmaceutically acceptable. Its examples include homopolymers, copolymers and the like of α-hydroxycarboxylic acids (lactic acid, glycolic acid and the like), hydroxydicarboxylic acids (malic acid and the like), hydroxytricarboxylic acids (citric acid and the like) and the like, of which a polylactic acid and a poly (lactic acid-glycolic acid) copolymer are desirable and a poly (lactic acid-glycolic acid) copolymer is particularly desirable. Also, these biodegradable polymers may be used alone or as a combination of two or more.

The spray-dried fine powder substantially comprises a physiologically active protein and saccharides, and the weight ratio of the physiologically active protein and the saccharides is preferably within the range of from about 0.01 to about 80% (w/w), more preferably from about 2 to about 50% (w/w) and most preferably from about 5 to about 20% (w/w). The active protein with less amount than this range would bear no substantial physiological activity and that with more than this range would bear no effect of the stabilization by saccharides.

The weight ratio of the spray-dried fine powder and the biodegradable polymer varies depending on the pharmacological effect and releasing period of physiologically active protein, but is within the range of from about 0.01 to about 20% (w/w), preferably from about 0.1 to about 10% (w/w). When the ratio is less than this range, releasing period would be considerably delayed and the releasing period would be possible to be shortened when that is more than this range.

The spray-dried fine powder of the invention can be obtained by, for example, spraying a mixed aqueous solution of a physiologically active protein and saccharides into the drying chamber of a spray drying apparatus using a nozzle, and thereby evaporating water. As the spray drying apparatus, a spray dryer can be exemplified. Examples of the nozzle to be used at the time of spray drying include a two fluid nozzle, a pressure nozzle, a rotary disc type and the like, and any of them can be used in accordance with the selection of physiologically active protein and saccharides.

In preparing the mixed aqueous solution, a water-soluble low molecular weight material may be dissolved in a physiologically active protein aqueous solution, or the physiologically active protein aqueous solution may be mixed with a water-soluble low molecular weight material aqueous solution. Also, as occasion demands, a pH adjusting agent (e.g., hydrochloric acid, sodium hydroxide and the like) and an osmotic pressure adjusting agent (e.g., sodium chloride and the like) may be added in case that they do not spoil the effect of the invention. In that case, concentration of the physiologically active protein in the mixed aqueous solution is not particularly limited with the proviso that it is within such a range that the physiologically active protein is dissolved, but is from about 0.1 to about 20 mg/ml, preferably from about 1 to about 10 mg/ml, more preferably from about 1 to about 8 mg/ml. Also, concentration of the saccharides in the mixed aqueous solution is not particularly limited with the proviso that it is within such a range that the saccharides are dissolved and the physiologically active protein does not precipitate, but is from about 1 to about 150 mg/ml, preferably from about 10 to about 100 mg/ml, more preferably from about 20 to about 70 mg/ml.

Blower inlet temperature of the blowing chamber of spray drying chamber at the time of spray drying is within the range of from about 20 to about 150°C, preferably from about 40 to about 120°C, more preferably from about 50 to about 100°C. Also, the blower outlet temperature is within the range of from about 0 to about 80°C, preferably from about 10 to about 80°C, more preferably from about 20 to about 60°C.

Regarding the method for dispersing a spray-dried fine powder of a physiologically active protein and saccharides in a biodegradable polymer, it may be any one of a method in which a spray-dried fine powder is dispersed by adding an organic solvent to a mixture of the spray-dried fine powder and a biodegradable polymer and thereby dissolving the biodegradable polymer, a method in which a spray-dried fine powder is dispersed in a biodegradable polymer organic solvent solution, a method in which a biodegradable polymer is dissolved in a spray-dried fine powder organic solvent dispersion and a method in which an organic solvent dispersion of a spray-dried fine powder is mixed with an organic solvent solution of a biodegradable polymer. In dispersing a spray-dried fine powder in an organic solvent solution of a biodegradable polymer, a vortex mixer, homogenizer, sonicator or the like treatment may be carried out for the purpose of increasing its uniformity. Concentration of the biodegradable polymer in an organic solvent is preferably from about 10 to about 500 mg/ml, more preferably from about 50 to about 250 mg/ml.

The organic solvent to be used in the invention is not particularly limited with the proviso that it is a volatile solvent which does not dissolve the spray-dried fine powder but dissolves the biodegradable polymer. Illustrative examples of the organic solvent include acetonitrile, methylene chloride, chloroform, ethyl acetate, toluene and the like, of which acetonitrile and methylene chloride are desirable. These organic solvents may be used by mixing two or more of them.

Also, as occasion demands, an excipients or the like may be added thereto in case that it does not spoil the effect of the invention. As the excipient , pharmaceutically acceptable salts, surface active agents, saccharides, amino acids, organic acids, other water-soluble substances and the like can be exemplified, and their blending amount is from about 0.1 to about 20% (w/w), preferably from about 1 to about 10% (w/w), with reference to the weight of the biodegradable polymer. One or two or more of these fillers can be added.

Among the above excipients, those which do not dissolve in the organic solvent are added as powders, and average particle diameter of the excipients in that case is preferably 20 µm or less, more preferably 10 µm or less.

Illustrative examples of the salts include potassium L-glutamate, sodium L-glutamate, sodium edetate, sodium caprylate, sodium carbazochromsulfate, carboxymethylcellulose sodium, sodium citrate, calcium gluconate, sodium gluconate, magnesium gluconate, sodium m-sulfobenzoate, disodium hydrogenphosphate, sodium dihydrogenphosphate, dipotassium hydrogenphosphate, potassium dihydrogenphosphate, aluminum chloride, potassium chloride, calcium chloride, sodium chloride, sodium acetate, sodium carbonate, sodium bicarbonate and the like, examples of the saccharides include D-sorbitol, D-mannitol, inositol, xylitol, dextran, glucose, maltose, lactose, sucrose and the like, examples of the amino acids include methionine, aspartic acid, alanine, arginine, glycine, cysteine, taurine, histidine, phenylalanine, glutamic acid, lysine and the like, and examples of the other water-soluble substances include ascorbic acid, human serum albumin, sodium chondroitin sulfate, gelatin, gelatin hydrolysate, heparin sodium and the like.

In addition, surface active agents, organic acids and the like can also be added as the excipients which dissolve in the organic solvent. Illustrative examples of the surface active agents include sorbitan sesquioleate, sorbitan fatty acid ester, polyoxyethylene(160) polyoxypropylene(30) glycol, polyoxyethylene sorbitan monolaurate, polyoxyethylene castor oil, polyoxyethylene hydrogenated castor oil, polyoxyethylene hydrogenated castor oil 50, polyoxyethylene hydrogenated castor oil 60, polysorbate 20, polysorbate 80, macrogol 400, macrogol 4000, macrogol 600 and the like, and examples of the organic acids include oleic acid, thioglycollic acid, lactic acid and the like.

Regarding the method for solidifying a biodegradable polymer in an organic solvent, a method can be exemplified in which an O/O type emulsion is prepared by emulsifying a biodegradable polymer organic solvent solution by adding it to oils and the thus obtained emulsion is dried in liquid. The oils are not particularly limited with the proviso that they are oils which do not dissolve the spray-dried fine powder and biodegradable polymer and do not mix with organic solvents that dissolve the biodegradable polymer, and its preferred examples include silicone oil, soybean oil, sesame oil, cotton seed oil, mineral oil and the like, of which silicone oil and mineral oil are more desirable and silicone oil is particularly most desirable. These may be used by mixing two or more of them.

In addition, it is desirable to set volume of the oils to approximately from 1 to 1,000 times, particularly desirably approximately from 10 to 500 times, of the volume of the organic solvent solution of biodegradable polymer.

By removing the organic solvent in oils, incorporation into the biodegradable polymer becomes easy so that it has an advantage of attaining high inclusion ratio.

In addition, an agent to prevent aggregation can also be added to the oils. The agent is not particularly limited with the proviso that it inhibits aggregation of microspheres in oils, among agents sorbitan sesquioleate is desirable.

At the time of the emulsification, an appropriate apparatus such as a propeller mixer, a turbine type emulsifying machine, an ultrasonic dispersing apparatus, a high pressure emulsifying machine or the like can be used.

The drying in liquid can be carried out by a usual method (e.g., a vacuum method or the like) and it is desirable, for example, to evaporate the organic solvent under about 0.1 to about 50 mm/Hg while stirring the emulsion at about 10 to about 1,000 rpm using a propeller mixer, at a temperature of from 0 to about 25°C, more desirably at about 10 to about 20°C. The oils are removed with a mesh having a mesh size of from 0.1 to 20 µm. In removing oils, the oils can be efficiently removed by adding a solvent which mixes with oils. For example, the addition of isopropyl alcohol, ethanol or the like is effective. Also, if necessary, the organic solvent may be evaporated by further reducing the pressure to 1 mmHg or less. The temperature is preferably from 0 to about 30°C, more preferably from about 15 to about 25°C. In order to attain complete evaporation of the organic solvent, it is desirable to carry out the treatment under a reduced pressure for a period of from about 24 to about 60 hours, and a period of from about 36 to about 48 hours is a more desirable treating period. Removal of the oils can also be carried out by adding a solvent in which oils are dissolved but biodegradable polymers are not dissolved. Illustratively, the addition of n-hexane, isopropyl alcohol, ethanol, methanol or the like is effective.

If necessary, removal of the solvent in which oils are dissolved but biodegradable polymers are not dissolved is carried out by reducing the pressure to 1 mmHg or less for a period of from about 1 to about 2 hours. Microspheres having even particle diameter can be obtained by sieving the thus obtained microspheres through a mesh having a mesh size of from 100 to 500 µm, preferably through a mesh having a mesh size of from 150 to 300 µm. Also, if necessary, the organic solvent remained in the thus obtained microspheres can be further removed by heating the microspheres. The heating may be carried out under a reduced pressure. By carrying out a series of these operations, it is possible to obtain microspheres having an average particle diameter of from about 20 µm to about 250 µm suited for injections.

Next, method for the production of the composition for sustained release injections of the invention is described.

In producing the composition of the invention, a physiologically active protein aqueous solution is mixed with a saccharides aqueous solution, and a spray-dried fine powder thereof is obtained using a spray dryer.
Separately, a biodegradable polymer (e.g., a poly (lactic acid-glycolic acid) copolymer (PLGA)) is dissolved in an appropriate organic solvent (e.g., acetonitrile) and then the physiologically active protein/saccharides spray-dried fine powder is added thereto and dispersed using Polytron. The spray-dried fine powder-dispersed biodegradable polymer solution is put into an oil (e.g., silicone oil) and stirred at 400 rpm to evaporate the solvent under a pressure of 10 mmHg for 30 minutes. By adding isopropyl alcohol or the like, microspheres are recovered using a sieve having a mesh size of 20 µm and then treated under a pressure of 0.1 mmHg or less for an appropriate period of time. After removing the oil by washing with hexane, hexane is removed by treating under a pressure of 0.1 mmHg or less for 1 hour. By recovering microspheres through a sieve of 250 µm in mesh size, the composition of the invention is obtained.

### Best Mode for Carrying Out the Invention

The following illustratively describes the invention with reference to examples, but the scope of the invention is not restricted thereby.

### [Method for measuring particle diameter]

Acetonitrile was added to each spray-dried fine powder and treated at 10,000 rpm for 1 minute using Polytron (KINEMATICA GmbH) to obtain dispersion. A part or entire portion of the dispersion was measured using a laser diffraction/scattering type particle size distribution measuring apparatus (HORIBA, LA-910).

### [Method for testing physiological activity]

### Preparation of samples for measuring activity in spray-dried powder

Each spray-dried fine powder was dissolved in 1/30 M phosphate buffer, pH 7.0, containing 7% (w/v) bovine serum albumin and then filtered using a 0.22 µm filter (MILLEX-GV: MILLIPORE). After measuring fluorescence intensity (excitation wavelength 495 nm, fluorescent wavelength 517 nm) using a fluorescence spectrometer (F-4500: HITACHI) and calculating concentration of FITC-labeled interferon-α-Con 1, this was used as a sample solution.

### Preparation of samples for measuring activity in microspheres

The PLGA was dissolved by adding acetone to spray-dried fine powder-containing microspheres, and after centrifugation (2,000 rpm, 10 minutes), the supernatant was discarded. By repeating this operation 4 times, the spray-dried fine powder was recovered. The thus recovered spray-dried fine powder was made into a sample solution by the aforementioned method.

### Method for measuring titer

Measurement of the titer of interferon-α-Con 1 was carried out by measuring antiviral activity (reference: Protein, Nucleic Acid and Enzyme, Supplement 25, 355 - 363, 1981). Regarding the cells, cells of a human amnion-derived strain (FL cell, ATCC CCL-62, a strain obtained from National Institute of Health of Japan or an equivalent preparation) and sindbis virus (a strain obtained from National Institute of Health of Japan) were used.

### [Dissolution test]

A 50 mg portion of PLGA microspheres containing fluorescein isothiocyanate (FITC)-labeled interferon-α-Con 1 were mixed with 10 ml of 1/30 M phosphate buffer (pH 7.0) and incubated at 60 strokes/min. in a water bath of 37°C. After a predetermined period of time, a portion of the supernatant was mixed with the same volume of 0.1 N sodium hydroxide containing 5% (w/v) of sodium dodecyl sulfate, and then released amount of interferon-α-Con 1 was calculated by measuring fluorescence intensity (excitation wavelength 495 nm, fluorescent wavelength 517 nm) using a fluorescence spectrometer (F-4500: HITACHI).

### [Measurement of encapsulation ratio]

A 20 mg portion of PLGA microspheres containing FITC-labeled interferon-α-Con 1 were dissolved by adding 10 ml of dimethyl sulfoxide. After adding 10 ml of 0.1 N sodium hydroxide containing 5% (w/v) sodium dodecyl sulfate, inclusion ratio of interferon-α-Con 1 was calculated by measuring fluorescence intensity (excitation wavelength 495 nm, fluorescent wavelength 517 nm) using a fluorescence spectrometer (F-4500: HITACHI).

### Example 1

### [FITC-labeling of interferon-α-Con 1]

A 40 ml portion of interferon-α-Con 1 aqueous solution (19 mg/ml) was mixed with 150 ml of carbonate buffer of pH 9.5, and the mixture was adjusted to pH 9.5 with 0.1 N sodium hydroxide aqueous solution and ice-cooled. A 25 mg portion of fluorescein isothiocyanate was dissolved in the carbonate buffer and then added to ice-cooled interferon-α-Con 1 aqueous solution, and the mixture was stirred for a predetermined period of time. After adjusting to pH 7.0 by adding 1 N hydrochloric acid, FITC-labeled interferon-α-Con 1 was purified by a gel filtration using Sephadex G-25. The number of labeled FITC molecules per 1 mole of interferon-α-Con 1 was about 0.2.

### [Production of fluorescein isothiocyanate (FITC)-labeled interferon-α-Con 1/mannitol spray-dried fine powder]

An FITC-labeled interferon-α-Con 1 aqueous solution was mixed with a mannitol aqueous solution and adjusted to a final FITC-labeled interferon-α-Con 1 concentration of 5 mg/ml and a final mannitol concentration of 50 mg/ml. The spray drying conditions were blower inlet temperature of 95 to 105°C, liquid feeding rate of 6 g/min. and spray pressure of 2 kg/cm², the blower outlet temperature became of 35 to 45°C as a result. The recovered powder was stored in a closed container together with silica gel. When particle diameter of the spray-dried fine powder was measured, its average particle diameter was 4.7 µm, and the ratio of particles of 10 µm or less was 98.1%.

### [Production of PLGA microspheres containing FITC-labeled interferon-α-Con 1/mannitol spray-dried fine powder]

After dissolving 500 mg of PLGA in 2.5 ml of acetonitrile, 10 mg of the FITC-labeled interferon-α-Con 1/mannitol spray-dried fine powder was added thereto and dispersed by Polytron at 10,000 rpm for 1 minute. The spray-dried fine powder-dispersed polymer solution was poured into 500 ml of silicone oil and stirred at 400 rpm to evaporate acetonitrile under a pressure of 10 mmHg for 30 minutes. After adding 500 ml of isopropyl alcohol, microspheres were recovered using a sieve having a mesh size of 20 µm and then treated under a pressure of 0.1 mmHg or less for 36 hours. After removing silicone oil by washing with hexane, hexane was removed by treating under a pressure of 0.1 mmHg or less for 1 hour. By recovering microspheres through a sieve of 250 µm in mesh size, the physiologically active protein-containing composition for sustained release injection of the invention was obtained.

### Test Example 1

When calculated by the aforementioned encapsulation ratio measuring method, encapsulation ratio of the FITC-labeled interferon-α-Con 1/mannitol spray-dried fine powder in the microspheres prepared in Example 1 was 90%. Also, when dissolution of the FITC-labeled interferon-α-Con 1 from the microspheres was measured by the aforementioned dissolution test, its dissolution until initial 3 hours was 5% or less. Also, the dissolution was continued over 1 month or more.

Accordingly, it was verified that the physiologically active protein was efficiently encapsulated, initial burst was reduced and continuous release over a prolonged period of time was attained in the microspheres prepared by the production method of the invention using the spray-dried fine powder of the invention which used mannitol as the carrier.

### Example 2

### [Production of FITC-labeled interferon-α-Con 1/2-hydroxypropyl-β-cyclodextrin spray-dried fine powder]

An FITC-labeled interferon-α-Con 1 aqueous solution was mixed with a 2-hydroxypropyl-β-cyclodextrin aqueous solution and adjusted to a final FITC-labeled interferon-α-Con 1 concentration of 5 mg/ml and a final 2-hydroxypropyl-β-cyclodextrin concentration of 50 mg/ml. The spray drying conditions were blower inlet temperature of 95 to 105°C, liquid feeding rate of 6 g/min. and spray pressure of 2 kg/cm², the blower outlet temperature became 35 to 45°C as a result. The recovered powder was stored in a closed container together with silica gel. When particle diameter of the spray-dried fine powder was measured, its average particle diameter was 4.0 µm, and the ratio of particles of 10 µm or less was 99.4%.

### [Production of PLGA microspheres containing FITC-labeled interferon-α-Con 1/2-hydroxypropyl-β-cyclodextrin spray-dried fine powder]

After dissolving 500 mg of PLGA in 2.5 ml of acetonitrile, 10 mg of the FITC-labeled interferon-α-Con 1/2-hydroxypropyl-β-cyclodextrin spray-dried fine powder was added thereto and dispersed by Polytron at 10,000 rpm for 1 minute. The spray-dried fine powder-dispersed polymer solution was poured into 500 ml of silicone oil and stirred at 400 rpm to evaporate acetonitrile under a pressure of 10 mmHg for 30 minutes. After adding 500 ml of isopropyl alcohol, microspheres were recovered using a sieve having a mesh size of 20 µm and then treated under a pressure of 0.1 mmHg or less for 36 hours. After removing silicone oil by washing with hexane, hexane was removed by treating under a pressure of 0.1 mmHg or less for 1 hour. By recovering microspheres through a screen of 250 µm in mesh size, the physiologically active protein-containing composition for sustained release injections of the invention was obtained.

### Test Example 2

When measured by the aforementioned encapsulation ratio measuring method, encapsulation ratio of the FITC-labeled interferon-α-Con 1/2-hydroxypropyl-β-cyclodextrin spray-dried fine powder in the PLGA microspheres prepared in Example 1 was 85%. Also, when dissolution of the FITC-labeled interferon-α-Con 1 after soaking the PLGA microspheres in phosphate buffer of pH 7.0 was measured by the aforementioned dissolution test, its dissolution until initial 3 hours was 10% or less. Also, the dissolution was continued over 3 weeks or more.

Accordingly, it was verified that the physiologically active protein was efficiently encapsulated, initial burst was reduced and continuous release over a prolonged period of time was attained in the microspheres prepared by the production method of the invention using the spray-dried fine powder of the invention which used 2-hydroxypropyl-β-cyclodextrin as the carrier.

### Example 3

An interferon-α-Con 1 aqueous solution was mixed with a mannitol aqueous solution and adjusted to a final interferon-α-Con 1 concentration of 0.5 mg/ml and a final mannitol concentration of 50 mg/ml. The spray drying conditions were blower inlet temperature of 45 to 55°C, liquid feeding rate of 3 g/min. and spray pressure of 2 kg/cm², the blower outlet temperature was 23 to 28°C. Using the thus recovered interferon-α-Con 1/mannitol spray-dried fine powder, interferon-α-Con 1-containing PLGA microspheres were prepared by the same production method of Example 1.

### Test Example 3

### [Stability of interferon-α-Con 1 during the production process of interferon-α-Con 1-containing PLGA microspheres]

Using the interferon-α-Con 1/mannitol spray-dried fine powder-included PLGA microspheres shown in Example 3, stability of interferon-α-Con 1 during the production process was evaluated. Measurement of antiviral activity was carried out using the FL cell and sindbis virus. When compared with the activity value of interferon-α-Con 1 solution, activity of the interferon-α-Con 1 included in the PLGA microspheres was 103.5% so that it was confirmed that its stability was maintained during the production process without causing inactivation.

### Comparative Example 1

### [Production of consensus interferon/polyethylene glycol 4000 spray-dried fine powder]

A consensus interferon aqueous solution was mixed with a polyethylene glycol 4000 aqueous solution and adjusted to a final consensus interferon concentration of 0.2 mg/ml and a final polyethylene glycol 4000 concentration of 20 mg/ml. The spray drying conditions were blower inlet temperature of 47 to 52°C, liquid feeding rate of 3 g/min. and spray pressure of 2 kg/cm², and the blower outlet temperature became 26 to 31°C as a result. The thus recovered powder was stored in a closed container together with silica gel.

### [Stability of consensus interferon during the production process of consensus interferon-containing PLGA microspheres]

In order to evaluate its stability during the production process of consensus interferon/polyethylene glycol 4000 spray-dried fine powder, measurement of antiviral activity was carried out using the FL cell and sindbis virus. When compared with the activity value of consensus interferon solution, the consensus interferon/mannitol spray-dried fine powder shoed an activity of 31.2% so that its inactivation during the production process was confirmed. Thus, it was confirmed through the comparison with the example that inactivation of consensus interferon during the production process was avoided by saccharides.

### Comparative Example 2

### [Production of FITC-labeled consensus interferon/mannitol freeze-dried powder]

An FITC-labeled consensus interferon aqueous solution was mixed with a mannitol aqueous solution and freeze-dried after adjusting final concentration of the FITC-labeled consensus interferon to 3.9 mg/ml and that of mannitol to 50 mg/ml. When particle diameter of the freeze-dried powder was measured, its average particle diameter was 69.3 µm, and the ratio of particles of 10 µm or less was 3.3%.

### [Production of PLGA microspheres containing FITC-labeled consensus interferon/mannitol freeze-dried powder]

After dissolving 500 mg of PLGA in 2.5 ml of acetonitrile, 10 mg of the FITC-labeled consensus interferon/mannitol freeze-dried powder was added thereto and dispersed by Polytron at 10,000 rpm for 1 minute. The freeze-dried fine powder-dispersed polymer solution was poured into 500 ml of silicone oil and stirred at 400 rpm to evaporate acetonitrile under a pressure of 10 mmHg for 30 minutes. After adding 500 ml of isopropyl alcohol, microspheres were recovered using a sieve having a mesh size of 20 µm and then treated under a pressure of 0.1 mmHg or less for 36 hours. After removing silicone oil by washing with hexane, hexane was removed by treating under a pressure of 0.1 mmHg or less for 1 hour. By recovering microspheres through a sieve of 250 µm in mesh size, the physiologically active protein-containing composition for sustained release injections of the invention was obtained.

### [Evaluation of initial stage dissolution from PLGA microspheres containing FITC-labeled consensus interferon/mannitol freeze-dried powder]

When calculated by the aforementioned encapsulation ratio measuring method, encapsulation ratio of the FITC-labeled consensus interferon/mannitol freeze-dried powder in the PLGA microspheres was 122%. Also, when dissolution of the FITC-labeled consensus interferon from the microspheres was measured by the aforementioned dissolution test, its dissolution until initial 3 hours was 68%.

Accordingly, it was verified that the physiologically active protein was efficiently encapsulated, its initial burst was reduced and its continuous release over a prolonged period of time was attained in the physiologically active protein-containing composition for sustained release injections of the invention prepared using spray-dried fine powder. In addition, it was shown that microspheres showing excellent encapsulation ratio and initial stage dissolution can be obtained when those having a particle diameter of 10 µm or less in spray-dried fine powder are 90% or more.

### Example 4

### [Method for testing BMP-2]

### Preparation of samples for the measurement of activity in spray-dried fine powder

Each spray-dried fine powder was dissolved in a dilution buffer (0.01% Tween 80 glycine buffer, pH 4.5), and concentration of BMP-2 was measured by a micro Lowry method. Using Dulbecco's modified MEM medium (pH 7.4) containing 10% fetal bovine serum, a series of dilutions having BMP-2 concentrations of approximately from 0.488 to 1,380 ng/ml were prepared from the thus obtained dilution buffer solution of BMP-2 and used as sample solutions.

### Preparation of samples for the measurement of activity in microspheres

The PLGA was dissolved by adding acetone to spray-dried fine powder-containing microspheres, and after centrifugation (2,000 rpm, 10 minutes), the supernatant was discarded. By repeating this operation 4 times, the spray-dried fine powder was recovered.

The thus recovered spray-dried fine powder was made into sample solutions by the aforementioned method.

### Method for measuring titer

Measurement of the titer of BMP-2 was carried out by measuring alkaline phosphatase activity in a BMP-2 concentration-dependently accelerating cellular alkaline phosphatase activity (reference: *Endocrinology*, vol. 130, pp. 1318 - 1324, 1992). Regarding the cell, a bone marrow stroma cell, W 20 cell (a cell strain obtained from Genetics Institute, Inc. (presently Wyeth, Inc.)), was used.

### [Production of BMP-2/mannitol spray-dried fine powder]

A BMP-2 aqueous solution was mixed with a mannitol aqueous solution and adjusted to a final BMP-2 concentration of 0.5 mg/ml and a final mannitol concentration of 50 mg/ml. The spray drying conditions were blower inlet temperature 48 to 53°C, liquid feeding rate 3 g/min. and spray pressure 2 kg/cm², the blower outlet temperature became 26 to 31°C as a result. The recovered powder was stored in a closed container together with silica gel. When particle diameter of the spray-dried fine powder was measured, its average particle diameter was 4.9 µm, and the ratio of particles of 10 µm or less was 97.9%.

### Example 5

### [Production of BMP-2/mannitol/Tween 80 spray-dried fine powder]

A BMP-2 aqueous solution was mixed with a mannitol/Tween 80 aqueous solution and their final concentrations were adjusted to 0.5 mg/ml for BMP-2, 50 mg/ml for mannitol and 1 mg/ml for Tween 80. The spray drying conditions were blower inlet temperature of 48 to 53°C, liquid feeding rate of 3 g/min. and spray pressure of 2 kg/cm², the blower outlet temperature became 27 to 32°C as a result. The recovered powder was stored in a closed container together with silica gel. When particle diameter of the spray-dried fine powder was measured, its average particle diameter was 6.2 µm, and the ratio of particles of 10 µm or less was 90.2%.

### Example 6

### [Production of BMP-2/mannitol/glycine spray-dried fine powder]

A BMP-2 aqueous solution was mixed with a mannitol/glycine aqueous solution and their final concentrations were adjusted to 0.5 mg/ml for BMP-2, 45 mg/ml for mannitol and 5 mg/ml for glycine. The spray drying conditions were blower inlet temperature of 48 to 53°C, liquid feeding rate of 3 g/min. and spray pressure of 2 kg/cm², the blower outlet temperature became 26 to 31°C as a result. The recovered powder was stored in a closed container together with silica gel. When particle diameter of the spray-dried fine powder was measured, its average particle diameter was 4.6 µm, and the ratio of particles of 10 µm or less was 98.3%.

### Example 7

### [Production of BMP-2/2-hydroxypropyl-β-cyclodextrin spray-dried fine powder]

A BMP-2 aqueous solution was mixed with a 2-hydroxypropyl-β-cyclodextrin aqueous solution and their final concentrations were adjusted to 0.5 mg/ml for BMP-2 and 50 mg/ml for 2-hydroxypropyl-β-cyclodextrin. The spray drying conditions were blower inlet temperature of 48 to 53°C, liquid feeding rate of 3 g/min. and spray pressure of 2 kg/cm², the blower outlet temperature became 26 to 31°C as a result. The recovered powder was stored in a closed container together with silica gel. When particle diameter of the spray-dried fine powder was measured, its average particle diameter was 4.7 µm, and the ratio of particles of 10 µm or less was 98.3%.

### Example 8

### [Production of BMP-2/2-hydroxypropyl-β-cyclodextrin/Tween 80 spray-dried fine powder]

A BMP-2 aqueous solution was mixed with a 2-hydroxypropyl-β-cyclodextrin aqueous solution and their final concentrations were adjusted to 0.5 mg/ml for BMP-2, 50 mg/ml for 2-hydroxypropyl-β-cyclodextrin and 1 mg/ml for Tween 80. The spray drying conditions were blower inlet temperature of 47 to 52°C, liquid feeding rate of 3 g/min. and spray pressure of 2 kg/cm², the blower outlet temperature became 26 to 31°C as a result. The recovered powder was stored in a closed container together with silica gel. When particle diameter of the spray-dried fine powder was measured, its average particle diameter was 6.3 µm, and the ratio of particles of 10 µm or less was 90.1%.

### Example 9

### [Production of BMP-2/2-hydroxypropyl-β-cyclodextrin/glycine spray-dried fine powder]

A BMP-2 aqueous solution was mixed with a 2-hydroxypropyl-β-cyclodextrin/glycine aqueous solution and their final concentrations were adjusted to 0.5 mg/ml for BMP-2, 45 mg/ml for 2-hydroxypropyl-β-cyclodextrin and 5 mg/ml for glycine. The spray drying conditions were blower inlet temperature of 48 to 53°C, liquid feeding rate of 3 g/min. and spray pressure of 2 kg/cm², the blower outlet temperature became 25 to 30°C as a result. The recovered powder was stored in a closed container together with silica gel. When particle diameter of the spray-dried fine powder was measured, its average particle diameter was 5.8 µm, and the ratio of particles of 10 µm or less was 97.2%.

### Example 10

### [Production of BMP-2-containing spray-dried fine powder-included PLGA microspheres]

After dissolving 500 mg of PLGA in 2.5 ml of acetonitrile, 10 mg of the BMP-2/2-hydroxypropyl-β-cyclodextrin spray-dried fine powder was added thereto and dispersed by Polytron at 10,000 rpm for 1 minute. The spray-dried fine powder-dispersed polymer solution was poured into 500 ml of silicone oil and stirred at 400 rpm to evaporate acetonitrile under a pressure of 10 mmHg for 30 minutes. After adding 500 ml of isopropyl alcohol, microspheres were recovered using a sieve having a mesh size of 20 µm and then treated under a pressure of 0.1 mmHg or less for 36 hours. After removing silicone oil by washing with hexane, hexane was removed by treating under a pressure of 0.1 mmHg or less for 1 hour. By recovering microspheres through a sieve of 250 µm in mesh size, the physiologically active protein-containing composition for sustained release injection of the invention was obtained.

### Test Example 4

### [Stability of BMP-2 in BMP-2-containing spray-dried fine powder and PLGA microspheres]

In order to evaluate stability of BMP-2-containing spray-dried fine powder and BMP-2 incorporated in PLGA microspheres during the production process, biological activity measurement was carried out using W 20 cells. When compared with the activity value of BMP-2 solution, biological activity of BMP-2 incorporated in PLGA microspheres prepared by using the BMP-2-mannitol spray-dried fine powder on BMP-2-2-hydroxypropyl-β-cyclodextrin spray-dried fine powder maintained high values of 105.7% and 103.9%, respectively, thus confirming that its stability was maintained during the production process without causing inactivation.

### Example 11

### [Method for testing lysozyme chloride]

### Preparation of sample for the measurement of activity in spray-dried fine powder

A spray-dried fine powder was dissolved in purified water, and concentration of lysozyme chloride was measured by a micro Lowry method. Using a phosphate buffer of pH 6.2, this was diluted to a protein concentration of 1 µg/ml and used as a sample solution.

### Preparation of sample for the measurement of activity in microspheres

The PLGA was dissolved by adding acetone to spray-dried fine powder-containing microspheres, and after centrifugation (2,000 rpm, 10 minutes), the supernatant was discarded. By repeating this operation 4 times, the spray-dried fine powder was recovered. The thus recovered spray-dried fine powder was made into a sample solution by the aforementioned method.

### Method for measuring titer

The titer of lysozyme chloride was measured using increased substrate solubility in concentration-dependent manner by lysozyme chloride (reference: Non-official Drugs Standard, 1997, pp. 352 - 353). Regarding the substrate, dried cells of *Micrococcus lysodeikticus* were used.

### [Production of lysozyme chloride/mannitol spray-dried fine powder]

A lysozyme chloride aqueous solution was mixed with a mannitol aqueous solution and adjusted to a final lysozyme chloride concentration of 5 mg/ml and a final mannitol concentration of 50 mg/ml. The spray drying conditions were blower inlet temperature of 48 to 53°C, liquid feeding rate of 3 g/min. and spray pressure of 2 kg/cm², the blower outlet temperature became 24 to 30°C as a result. The recovered powder was stored in a closed container together with silica gel. When particle diameter of the spray-dried fine powder was measured, its average particle diameter was 4.9 µm, and the ratio of particles of 10 µm or less was 96.6%.

### Example 12

### [Production of lysozyme chloride /2-hydroxypropyl-β-cyclodextrin spray-dried fine powder]

A lysozyme chloride aqueous solution was mixed with a 2-hydroxypropyl-β-cyclodextrin aqueous solution and their final concentrations were adjusted to 10 mg/ml for lysozyme chloride and 50 mg/ml for mannitol. The spray drying conditions were blower inlet temperature of 49 to 50°C, liquid feeding rate of 3 g/min. and spray pressure of 2 kg/cm², the blower outlet temperature became 27 to 29°C as a result. The recovered powder was stored in a closed container together with silica gel. When particle diameter of the spray-dried fine powder was measured, its average particle diameter was 5.8 µm, and the ratio of particles of 10 µm or less was 90.9%.

### [Production of lysozyme chloride-containing spray-dried fine powder-included PLGA microspheres]

After dissolving 500 mg of PLGA in 2.5 ml of acetonitrile, 10 mg of the lysozyme chloride-containing spray-dried fine powder was added thereto and dispersed by treating with Polytron at 10,000 rpm for 1 minute. The spray-dried fine powder-dispersed polymer solution was poured into 500 ml of silicone oil and stirred at 400 rpm to evaporate acetonitrile under a pressure of 10 mmHg for 30 minutes. After adding 500 ml of isopropyl alcohol, microspheres were recovered using a sieve having a mesh size of 20 µm and then treated under a pressure of 0.1 mmHg or less for 36 hours. After removing silicone oil by washing with hexane, hexane was removed by treating under a pressure of 0.1 mmHg or less for 1 hour. By recovering microspheres through a sieve of 250 µm in mesh size, the physiologically active protein-containing composition for sustained release injections of the invention was obtained.

### Test Example 5

### [Stability of lysozyme chloride during production process of lysozyme chloride-containing PLGA microspheres]

In order to evaluate stability of lysozyme chloride solution, lysozyme chloride-containing spray-dried fine powder and lysozyme chloride incorporated in PLGA microspheres during the production process, activity measurement was carried out using dried cells of *Micrococcus lysodeikticus*. When compared with the activity value of lysozyme chloride solution, the activity of lysozyme chloride in PLGA microspheres prepared using the lysozyme chloride/mannitol spray-dried fine powder was 90.1%, and the activity of lysozyme chloride in PLGA microspheres prepared using the lysozyme chloride/2-hydroxypropyl-β-cyclodextrin spray-dried fine powder was 92.6%. Since both cases showed the activity of not lower than 90%, it was confirmed that the stability was maintained during the production process without causing inactivation.

### Industrial Applicability

According to the invention, spray drying can be carried out without employing a high temperature by the use of saccharides, so that denaturation and inactivation of physiologically active protein by heat can be avoided and a protein fine powder having controlled particle diameter can be prepared markedly easily. In addition, the use of the protein fine powder which maintains a physiological activity and has a sufficiently controlled particle diameter renders possible uniform dispersion of the powder in a biodegradable polymer solution, maintenance of the physiological activity of the incorporated physiologically active protein and attainment of high encapsulation ratio in microspheres and inhibition of initial burst.

Thus, the invention rendered possible incorporation of spray-dried fine powder in sustained release microspheres with a high encapsulation ratio and also rendered possible attainment of the inhibition of initial burst and continuous release from microspheres, and thereby rendered possible provision of a composition for sustained release injections having high productivity of physiologically active protein which is generally considered to be difficult in keeping its physiological activity during the drug preparation process.

## Claims

1. A physiologically active protein-containing composition for sustained release injections, which comprises a spray-dried fine powder comprising a physiologically active protein and a saccharide or the like,
wherein particles having a particle diameter of 10 µm or less occupy 90% or more of the spray-dried fine powder, and the spray-dried fine powder is uniformly dispersed in the matrix of a biodegradable polymer.

2. The physiologically active protein-containing composition for sustained release injections described in claim 1, wherein 50% or more of a spray-dried fine powder has a particle diameter of 6 µm or less.

3. The physiologically active protein-containing composition for sustained release injections described in claim 2, wherein the saccharide is mannitol and/or a cyclodextrin or the like.

4. The physiologically active protein-containing composition for sustained release injections described in claim 3, wherein the cyclodextrin is 2-hydroxypropyl-β-cyclodextrin or the like.

5. The physiologically active protein-containing composition for sustained release injections described in claim 4, wherein the biodegradable polymer is a poly (lactic acid-glycolic acid) copolymer.

6. The physiologically active protein-containing composition for sustained release injections described in claim 5, wherein the physiologically active protein is an interferon or the like.

7. The physiologically active protein-containing composition for sustained release injections described in claim 6, wherein the interferon is interferon-α-Con 1.

8. The physiologically active protein-containing composition for sustained release injections described in claim 7, wherein the composition is microsphere.

9. The physiologically active protein-containing composition for sustained release injections described in claim 8, wherein average particle diameter of the microsphere is set to from about 20 to about 250 µm.

10. The physiologically active protein-containing composition for sustained release injections described in claim 9, wherein weight ratio of the physiologically active protein and the saccharide is from about 0.01 to about 80% (w/w).

11. The physiologically active protein-containing composition for sustained release injections described in claim 9, wherein concentration of the spray-dried fine powder in the biodegradable polymer is from about 0.01 to about 20% (w/w).

12. A physiologically active protein-containing composition for sustained release injections, which comprises a physiologically active protein and a saccharide as substantial composing components, and is prepared by dispersing a spray-dried fine powder, 90% or more of which has a particle diameter of 10 µm or less, in an organic solvent solution of a biodegradable polymer, and removing said organic solvent in an oil.

13. The physiologically active protein-containing composition for sustained release injections described in claim 12, wherein 50% or more of a spray-dried fine powder has a particle diameter of 6 µm or less.

14. The physiologically active protein-containing composition for sustained release injections described in claim 13, wherein the saccharide is mannitol and/or a cyclodextrin or the like.

15. The physiologically active protein-containing composition for sustained release injections described in claim 14, wherein the cyclodextrin is 2-hydroxypropyl-β-cyclodextrin.

16. The physiologically active protein-containing composition for sustained release injections described in claim 15, wherein the biodegradable polymer is a poly (lactic acid-glycolic acid) copolymer.

17. The physiologically active protein-containing composition for sustained release injections described in claim 16, wherein the physiologically active protein is an interferon or the like.

18. The physiologically active protein-containing composition for sustained release injections described in claim 17, wherein the interferon is interferon-α-Con 1.

19. The physiologically active protein-containing composition for sustained release injections described in claim 18, wherein the oil is silicone oil, soybean oil, sesame oil, cotton seed oil or mineral oil.

20. The physiologically active protein-containing composition for sustained release injections described in claim 19, wherein the oil is silicone oil.

21. The physiologically active protein-containing composition for sustained release injections described in claim 20, wherein the composition is microsphere.

22. The physiologically active protein-containing composition for sustained release injections described in claim 21, wherein average particle diameter of the microsphere is from about 20 to about 250 µm.

23. The physiologically active protein-containing composition for sustained release injections described in claim 9, wherein weight ratio of the physiologically active protein and the saccharide is from about 0.01 to about 80% (w/w).

24. The physiologically active protein-containing composition for sustained release injections described in claim 22, wherein concentration of the spray-dried fine powder in the biodegradable polymer is 10% (w/w) or less.

25. A method for producing a physiologically active protein-containing composition for sustained release injections, which comprises a physiologically active protein and a saccharide as substantial composing components, and which comprises using dispersion of a spray-dried fine powder, 90% or more of which being occupied by particles having a particle diameter of 10 µm or less, in an organic solvent solution of a biodegradable polymer, and removing said organic solvent in an oil.

26. The method for producing a physiologically active protein-containing composition for sustained release injections described in claim 25, wherein 50% or more of a spray-dried fine powder has a particle diameter of 6 µm or less.

27. The method for producing a physiologically active protein-containing composition for sustained release injections described in claim 26, wherein the saccharide is mannitol and/or a cyclodextrin or the like.

28. The method for producing a physiologically active protein-containing composition for sustained release injections described in claim 27, wherein the cyclodextrin is 2-hydroxypropyl-β-cyclodextrin.

29. The method for producing a physiologically active protein-containing composition for sustained release injections described in claim 28, wherein the biodegradable polymer is a poly (lactic acid-glycolic acid) copolymer.

30. The method for producing a physiologically active protein-containing composition for sustained release injections described in claim 29, wherein the physiologically active protein is an interferon or the like.

31. The method for producing a physiologically active protein-containing composition for sustained release injections described in claim 30, wherein the interferon is interferon-α-Con 1.

32. The method for producing a physiologically active protein-containing composition for sustained release injections described in claim 31, wherein the oil is silicone oil, soybean oil, sesame oil, cotton seed oil or mineral oil.

33. The method for producing a physiologically active protein-containing composition for sustained release injections described in claim 32, wherein the oil is silicone oil.

34. The method for producing a physiologically active protein-containing composition for sustained release injections described in claim 33, wherein the composition is microsphere.

35. The method for producing a physiologically active protein-containing composition for sustained release injections described in claim 34, wherein average particle diameter of the microsphere is from about 20 to about 250 µm.

36. The method for producing a physiologically active protein-containing composition for sustained release injections described in claim 35, wherein concentration of the spray-dried fine powder in the biodegradable polymer is 10% (w/w) or less .

37. The method for producing a physiologically active protein-containing composition for sustained release injections described in claim 9, wherein weight ratio of the physiologically active protein and the saccharide is from about 0.01 to about 80% (w/w).
